# EUROPEAN PATENT APPLICATION

(11) **EP 4 822 015 A1**
(43) Date of publication of application: **14.10.2026**
(21) Application number: 24933815.3
(22) Date of filing: 27.11.2024
(51) Int. Cl.: C12N 5/077, A61K 35/33, A61P 19/02, A61P 1/16, A61P 29/00

(54) **METHOD FOR INHIBITING INFLAMMATORY RESPONSE BY USING FIBROBLAST**

(30) Priority: 03.04.2024 CN 202410400341
(71) Applicant: Fibrox Therapeutics (Shanghai) Co., Ltd., Shanghai 201203 (CN)
(72) Inventor: ZHANG, Qin, Shanghai 200135 (CN); ZHOU, Shenao, Shanghai 200135 (CN); ZHOU, Li, Shanghai 200135 (CN); YANG, Hao, Shanghai 200135 (CN); WEN, Mengnan, Shanghai 200135 (CN)
(74) Representative: Santarelli
(86) International application number: PCT/CN2024/134921
(87) International publication number: WO 2025/208891

(57) **Abstract**

The present invention relates to the technical field of biology, and particularly relates to a method for inhibiting an inflammatory response by using a fibroblast. In the present invention, a fibroblast, which is obtained by digesting normal foreskin tissue by means of clostridiopeptidase A to isolate a cell as a seed cell and culturing the seed cell in vitro by using a serum-free medium, shows positive expression of CD105, CD90, CD73, COL1A1, and FAP, with expression rates all exceeding 80%, and negative expression of CD36, CD39, CD45, and HLA class II molecules, with expression rates below 5%. The fibroblast can regulate an immune response by secreting TGFβ, inhibiting proliferation of PBMCs, thereby inhibiting the inflammatory response, and has been verified in an animal experiment to have the ability to repair knee osteoarthritis caused by meniscus injury. Therefore, the fibroblast can be used for preventing and treating inflammatory lesions such as osteoarthritis.

## Description

### Technical Field

The present invention belongs to the technical field of biomedicine, and specifically relates to a method for inhibiting an inflammatory response by using fibroblasts.

### Background of the Invention

Osteoarthritis is a common chronic inflammatory lesion that can affect joint tissues throughout the body. The World Health Organization (WTO) predicts that osteoarthritis will become the fourth leading cause of disability by 2020. Knee osteoarthritis refers to a chronic osteoarthropathy caused by articular cartilage degeneration and osteoproliferation of the knee joint; clinically, it is mainly manifested as knee joint pain and varying degrees of functional impairment, with joint swelling and effusion in some patients, seriously affecting the quality of life of patients; X-ray manifestations include narrowing of the joint space, osteophyte formation at joint margins, and subchondral bone sclerosis and cystic change. Knee osteoarthritis is also known as proliferative arthritis of the knee joint, degenerative arthritis and osteoarthropathy, occurs frequently in middle-aged and elderly people, may also occur in young people, may occur unilaterally or bilaterally, and is more common in women than in men. Among people over 55 years of age, about 60% show manifestations of knee osteoarthritis on X-ray films, of whom 35%-50% have clinical manifestations. Generally, articular cartilage begins to degenerate rapidly after the age of 30, and as age increases and use becomes frequent, the cartilage gradually becomes thinner and may even rupture and develop defects; in addition, obesity is an important risk factor in the occurrence and development of knee osteoarthritis; whenever body weight increases by 5%, the force borne by the knee joint increases by 35%. According to WHO data, there are about 355 million patients with knee osteoarthritis worldwide, and it is expected to affect as many as 25% of the population by 2040.

Knee osteoarthritis can lead to joint pain, deformity and impairment of motor function, thereby increasing the incidence of cardiovascular events and overall mortality. Existing treatment strategies are to relieve pain, improve joint function and delay disease progression. At present, commonly used clinical treatment means include the following four types:

The first is basic exercise, physical therapy, mobility assistance support and the like, but these can only temporarily relieve discomfort symptoms of the knee joint and cannot improve or halt disease progression of knee osteoarthritis. The second is drug therapy, mostly including nonsteroidal anti-inflammatory drug (NSAIDs), opioids, corticosteroid injection and the like. They have many adverse reactions and certain dependence and drug resistance. The third is intra-articular injection of hyaluronic acid, glucocorticoid and the like; although it can relieve pain and improve joint function, it also aggravates cartilage wear, affects calcium and phosphorus absorption, and reduces pain sensitivity. The 2nd edition of the AAOS evidence-based guideline for knee osteoarthritis in 2013 strongly does not recommend the use of sodium hyaluronate. The fourth is joint replacement. The joint replacement can improve patient symptoms, but there are various complications such as prosthesis loosening, infection and periprosthetic fracture.

In addition to the four clinically commonly used treatment methods, cell therapy is currently also applied to treat osteoarthritis, mainly using mesenchymal stem cell; mesenchymal stem cells (MSCs) have the abilities of self-replication, multidirectional differentiation and immunoregulation. At present, the most studied MSCs are mainly derived from bone marrow; however, because bone marrow-derived MSCs may have a high possibility of viral contamination and their cell number, expansion and differentiation abilities show an obvious decreasing trend with increasing age, problems such as low cell survival rate and cell leakage in the hypoxic and inflammatory environment of osteoarthritic joints possibly leading to neoplasm formation are the main challenges currently faced by MSCs therapy.

Liver failure is severe liver damage caused by various factors, resulting in severe impairment of functions such as synthesis, metabolism, detoxification and biotransformation of the liver, causing accumulation of a large amount of toxic substances in the body and thereby producing a group of clinical syndromes mainly manifested by coagulation dysfunction, hyperbilirubinemia, hepatic encephalopathy and ascites. Because the pathogenesis of liver failure has not yet been fully clarified, there is a lack of sensitive and standard diagnosis and treatment systems clinically, and there are no specific drugs or means for treatment; treatment of liver failure remains a difficult problem worldwide.

Traditional comprehensive medical treatment is the basis of treatment for liver failure; although monitoring techniques and supportive therapy are continuously improving, the mortality rate of patients remains as high as 60%-80%, and among them acute liver failure has an extremely high mortality rate; liver transplantation is currently recognized as the only effective method for treating patients with liver failure. However, due to shortage of donor livers, long waiting times and high transplantation costs, the number of patients who can actually receive liver transplantation treatment is very limited. In addition, patients with liver failure themselves face various problems during the perioperative period of liver transplantation, including severely impaired liver function before surgery, a long waiting time for a donor liver, and the possibility of various complications after transplantation, all of which are directly related to the short-term efficacy of transplantation and long-term survival rate of patients. Therefore, seeking new, rapid and effective treatment methods is the focus of liver failure research, and how to help patients with liver failure safely pass through the preoperative waiting period or recover their own liver function without needing liver transplantation, and effectively cope with complex complications after transplantation, is a focus of medical attention.

### Summary of the Invention

Based on the above needs, the present invention provides a fibroblast, which is obtained by digesting normal foreskin tissue by means of clostridiopeptidase A to isolate a cell as a seed cell and culturing the seed cell in vitro by using a serum-free medium, shows positive expression of CD105, CD90, CD73, COL1A1, and FAP, with expression rates all exceeding 80%, and negative expression of CD36, CD39, CD45, and HLA class II molecules, with expression rates below 5%. The fibroblast can regulate an immune response by secreting TGFβ, inhibiting proliferation of PBMCs, thereby inhibiting the inflammatory response, and has been verified in an animal experiment to have the ability to repair knee osteoarthritis caused by meniscus injury and verified in an animal model to have the ability to treat acute-on-chronic liver failure induced by CCL4 (carbon tetrachloride) and LPS (lipopolysaccharide).

The present invention provides a method for preparing a fibroblast, comprising the following steps:
S1: using a foreskin tissue as a raw material, and obtaining a primary fibroblast from a cell isolated by digestion with clostridiopeptidase A;
S2: performing in vitro culture of the primary fibroblast using a serum-free medium;
S3: obtaining the fibroblast, wherein the fibroblast shows positive expression of CD105, CD90, CD73, COL1A1 and FAP, with expression rates all exceeding 80%, negative expression of CD36, CD39, CD45 and HLA class II molecules, with expression rates below 5%, and can secrete TGF-β.

Preferably, the foreskin tissue is foreskin tissue discarded from clinical circumcision surgery, preferably a surgical sample derived from a male patient not more than 16 years of age, and the patient has no infectious viral infection as determined by medical examination.

More specifically, the method of S1 is as follows:
S1-1: washing and disinfecting the foreskin tissue with normal saline, then adding neutral protease A, incubating and then washing the foreskin tissue with pre-cooled normal saline, mechanically separating the dermal layer and the epidermal layer of the tissue, removing the epidermal layer, cutting the dermal layer into pieces as finely as possible, washing the pieces with pre-cooled normal saline, and adding clostridiopeptidase A and incubating same to obtain the primary fibroblast;
More specifically, the operation is as follows: washing and disinfecting the foreskin tissue with normal saline, adding neutral protease A to a final concentration of 2 DMC U/mL, incubating the foreskin tissue at 37°C for 16-20 h, washing the foreskin tissue 1-2 times with pre-cooled normal saline, separating the dermal layer and the epidermal layer of the tissue using sterilized forceps, removing the epidermal layer, cutting the dermal layer as finely as possible into 0.5-2 mm pieces, washing the pieces 1-2 times with pre-cooled normal saline, transferring the pieces to a 50 ml centrifuge tube, adding clostridiopeptidase A to a final concentration of 0.1 PZ U/mL, and incubating the pieces at 37°C for 2-3 h;
S1-2: screen sorting the cell suspension obtained in S1-1l through a 70 µm cell strainer with the aid of normal saline, collecting the cell suspension under the cell strainer, centrifuging to discard the supernatant, washing by centrifugation with serum-free medium, and resuspending in the serum-free medium to obtain a primary cell suspension;
A further specific operation is as follows: screen sorting the cell suspension through a 70 µm cell strainer with the aid of normal saline, collecting the cell suspension under the cell strainer, centrifuging at 1000 rpm for 5 minutes, discarding the supernatant, washing 1-2 times by centrifugation with serum-free medium, and resuspending in the serum-free medium to obtain the primary cell suspension;
S1-3: screen sorting the primary cell suspension through a 40 µm cell strainer with the aid of normal saline, collecting the cell suspension harvested from filtration through the cell strainer to complete the screen sorting, centrifuging the resulting filtrate and discarding the supernatant to obtain the primary fibroblast.

In addition, in specific embodiments, the method of S2 is as follows:
expanding the primary fibroblast in the serum-free medium until the cell confluence reached not less than 80%, then digesting the primary fibroblast with trypsin digestion solution, and subsequently subculturing the primary fibroblast using the serum-free medium to obtain the fibroblast;
preferably, the serum-free medium is high-glucose DMEM supplemented with hydrocortisone, bFGF and human platelet lysate.

More specifically, the method of S2 is as follows: digesting the primary fibroblast with the trypsin digestion solution for 1-5 minutes; the concentration of hydrocortisone is 5-25 ng/mL, the concentration of bFGF is 5-30 ng/mL, and the concentration of human platelet lysate is 4-10%; preferably, the concentration of hydrocortisone is 10-20 ng/mL, the concentration of bFGF is 15-25 ng/mL, and the concentration of human platelet lysate is 6-9%.

The present invention further provides the fibroblast obtained by the method, wherein the fibroblast shows positive expression of CD105, CD90, CD73, COL1A1 and FAP, with expression rates all exceeding 80%, negative expression of CD36, CD39, CD45 and HLA class II molecules, with expression rates below 5%, and can secrete TGF-β.

The present invention further provides use of the fibroblast in the preparation of a medicament for treating an inflammatory disease, preferably the inflammatory disease is local inflammation of osteoarthritis or acute liver failure.

Specifically, the disease is osteoarthritis and acute liver failure.

Therefore, the present invention further provides a pharmaceutical composition containing the fibroblast as an active ingredient. Specifically, the pharmaceutical composition is a dosage form suitable for injection, for example a dosage form for intra-articular injection, intramuscular injection and intravenous injection. When used for treating osteoarthritic inflammatory disease, a microinjection needle may be used to directly administer the cell suspension by intra-articular injection, with an injection volume of less than 1 mL.

The present invention uses cells obtained by digestion of normal foreskin tissue and isolation as seed cells, and performs in vitro culture using a serum-free medium, wherein hydrocortisone, bFGF and human platelet lysate are added to the medium. bFGF (fibroblast growth factor) regulates proliferation of fibroblasts, can significantly improve the proliferation rate of fibroblasts and improve expression of fibroblast collagen; hydrocortisone can significantly shorten the doubling time of fibroblasts, and human platelet lysate, as a serum substitute, can promote cell adherent proliferation. The fibroblast obtained by such culture shows positive expression of CD105, CD90, CD73, COL1A1, and FAP, with expression rates all exceeding 80%, and negative expression of CD36, CD39, CD45, and HLA class II molecules, with expression rates below 5%. The fibroblast can regulate an immune response by secreting TGFβ, inhibiting proliferation of PBMCs, thereby inhibiting the inflammatory response, and has been verified in an animal experiment to have the ability to repair knee osteoarthritis caused by meniscus injury and verified in an animal model to have the ability to treat acute-on-chronic liver failure induced by CCL4 (carbon tetrachloride) and LPS (lipopolysaccharide). Therefore, the fibroblast can be used for the prevention and treatment of diseases such as osteoarthritis and liver failure.

Treatment of osteoarthritis and liver failure with human fibroblast injection meets urgent market needs, and the present invention has the following advantages: ① Avoiding ethical restrictions: Compared with mesenchymal stem cell derived from embryonic stem cells or bone marrow stem cells, foreskin is medical waste, has no ethical restrictions, is widely available and easy to obtain, and causes no injury to donors; ② Helping patients with liver failure pass through the acute stage; ③ Simple transplantation method and reduced patient suffering: Cells are directly injected into the joint cavity or administered by intravenous injection, the surgical difficulty is low, the wound caused to the patient is small, and patient suffering is reduced; ④ High safety and low likelihood of causing infection: Fibroblasts have no immunogenicity and no pathogenic microbial infection, and adverse reactions such as postoperative infection in patients are not prone to occur; ⑤ No need for matching and no immune rejection: Fibroblasts have no immunogenicity, matching is not required for injection, and patients do not need to use an immunosuppressant; ⑥ Stable quality and definite effect: Fibroblast injection solution is managed and prepared as a drug, has a large output and small inter-batch differences in effect, and can be supplied to patients for repeated use at any time. The fibroblast provided by the present invention can be subjected to allogeneic reinfusion and large-scale expansion in vitro. Therefore, the present invention has extremely great application prospects and application value.

### Brief Description of the Drawings

FIG. 1 shows a flow chart of fibroblast isolation and culture.
FIG. 2 shows a morphology image of fibroblasts.
FIG. 3A to FIG. 3I show result diagrams respectively showing, in flow cytometric detection, that the positive rates of positive cell marker CD90, FAP, COL1A1, CD105 and CD73 are greater than 80%, and the positive rates of negative cell marker CD36, CD39, CD45 and HLA-DRPQ are less than 5%.
FIG. 4A shows the inhibition of PBMCs proliferation by fibroblasts after co-culture of donor 23019 cells with activated PBMC cells.
FIG. 4B shows the inhibition of PBMCs proliferation by fibroblasts after co-culture of donor 23021 cells with activated PBMC cells.
FIG. 4C shows the inhibition of PBMCs proliferation by fibroblasts after co-culture of MSC cells with activated PBMC cells.
FIG. 4D shows the status of PBMC cells of unactivated PBMC control as a negative group.
FIG. 4E shows the status of PBMC cells of activated PBMCs as a positive control.
FIG. 5 shows secretion of TGFβ by fibroblasts.
FIG. 6 shows secretion of CXCL12 by fibroblasts.
FIG. 7A shows staining of left knee joint tissue in a normal group.
FIG. 7B shows staining of left knee joint tissue in an experimental group.
FIG. 7C shows staining of left knee joint tissue in a model group.
FIG. 7D shows staining of right knee joint tissue in a normal group.
FIG. 7E shows staining of right knee joint tissue in an experimental group.
FIG. 7F shows staining of right knee joint tissue in a modelling group.
FIG. 8A shows glutamic-pyruvic transaminase detection results.
FIG. 8B shows glutamic-oxaloacetic transaminase detection results.
FIG. 8C shows survival curves of animals in the three groups.
FIG. 8D shows pathological staining after liver sampling from animals in the normal group.
FIG. 8E shows pathological staining after liver sampling from animals in the modelling group.
FIG. 8F shows pathological staining after liver sampling from animals in the experimental group.

### Detailed Description of Embodiments

The present invention is further described below through specific examples, in order to better understand the present invention, but the examples do not constitute a limitation on the present invention.

Example 1. In vitro culture of seed cells using a serum-free medium to obtain primary cells

### 1. Statement on properties of starting tissue and legality of source:

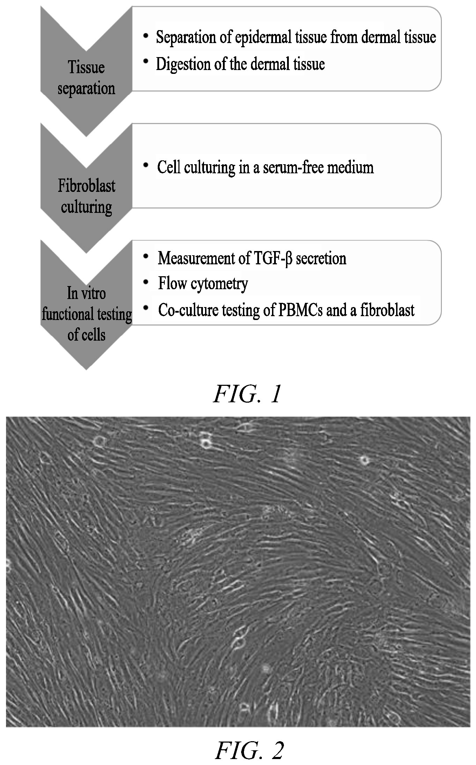

As shown in the flowchart of fibroblast isolation and culture in FIG. 1, in the present invention, cells obtained by digestion of normal foreskin tissue with clostridiopeptidase A and isolation were used as seed cells, and were cultured in vitro using a serum-free medium to obtain fibroblasts; the fibroblasts positively expressed CD105, CD90, CD73, COL1A1 and FAP, and negatively expressed CD36, CD39, CD45 and HLA class II molecules.

In this example, the foreskin tissue excised in circumcision was normal skin tissue and was a surgical sample derived from a male patient not more than 16 years of age; the patient had no infectious viral infection as determined by medical examination, the patient was fully informed before surgery of the purpose of obtaining the surgical sample, and the patient himself or his guardian signed an informed consent form.

### 2. Preparation of primary cells:

First, after the foreskin tissue was washed and disinfected with normal saline, neutral protease A was added at a final concentration of 2 DMC U/mL, incubation was performed at 37°C for 16-20 h, washing was performed 1-2 times with pre-cooled normal saline, the dermal layer and epidermal layer of the tissue were separated with sterile forceps, the epidermal layer was removed, the dermal layer was cut as finely and as small as possible to 0.5-2 mm pieces, washing was performed 1-2 times with pre-cooled normal saline, the pieces were transferred into a 50 ml centrifuge tube, and clostridiopeptidase A was added at a final concentration of 0.1 PZ U/mL followed by incubation at 37°C for 2-3 h.

Then, the cell suspension was subjected to screen sorting with the assistance of normal saline using a 70 µm cell strainer, the filtrate was collected, and mucus and undigested tissue that had not been filtered through and remain on the cell strainer were removed. Then, the cell suspension under the cell strainer was centrifuged at 1000 rpm for 5 minutes, the supernatant was discarded, washing was performed by centrifugation 1-2 times with the serum-free medium, and the cells were resuspended in the serum-free medium to obtain a primary cell suspension.

Then, the primary cell suspension was subjected to screen sorting with the assistance of normal saline using a 40 µm cell strainer, the cell suspension filtered and harvested under the cell strainer was collected to complete screen sorting, and after the obtained filtrate was centrifuged and the supernatant was removed, primary cells were obtained.

Example 2. In vitro culture of a cell suspension of primary fibroblasts using a serum-free medium to obtain target fibroblasts.

The specific steps of in vitro culture were as follows:
The primary fibroblasts were seeded in a 6-well cell culture plate at a seeding area of 15000 cells/cm², 2 millilitres of serum-free medium was added to each well for expansion culture until the cell confluence was not less than 80%, digestion was performed for 1-5 minutes using 1 mL of recombinant trypsin (TrypLE^{™}) digestion solution, and then subculture was continued using serum-free medium. The serum-free medium was high-glucose DMEM supplemented with 15 ng/mL hydrocortisone, 20 ng/mL bFGF and 7.5% human platelet lysate.

The primary cells were seeded in a 6-well plate at a seeding area of 15000 cells/cm², 2 millilitres of serum-free medium was added to each well for expansion culture until the cell confluence was not less than 80%, digestion was performed for 1-5 minutes using a recombinant trypsin digestion solution, and then subculture was continued using the serum-free medium, so that continuous passage can be achieved. The morphology of the fibroblasts obtained in this example is shown in FIG. 2.

Example 3. Related detection and analysis of fibroblasts
1. Flow cytometric detection of surface antibodies was performed on the obtained fibroblasts.

After the obtained fibroblast culture medium was aspirated and discarded, rinsing was performed with sterile PBS buffer, and then digestion was performed using a recombinant trypsin digestion solution, followed by centrifugation to collect a precipitate; 100 microliters of staining buffer was added to the precipitate to resuspend the cells into a flow tube, flow antibodies to be tested were respectively added, incubation was performed for 30 minutes, and then each tube was resuspended with 400 microliters of staining buffer for flow cytometric detection of surface antibodies.

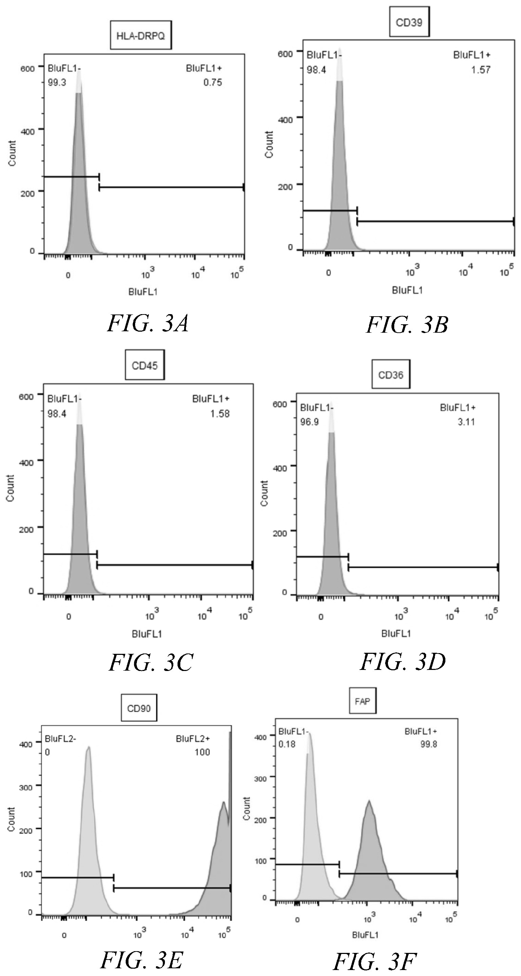

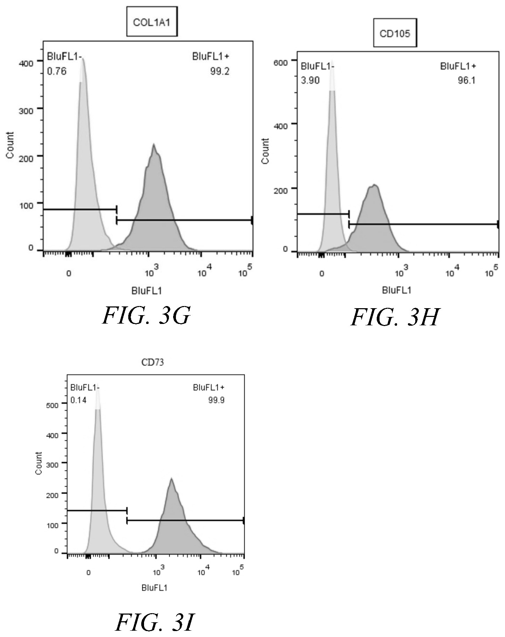

The flow cytometric detection results are shown in FIG. 3A to FIG. 3I. The results showed that positive cell marker with positive rates greater than 70% includes: CD90, FAP, COL1A1, CD105 and CD73.

2. In an in vitro experiment of fibroblasts, PBMCs proliferation can be inhibited.

Activated PBMC cells and fibroblasts were mixed and co-cultured in a 24-well culture plate, and after culture for 96 hours, proliferation of PBMC cells was observed; activated PBMC cells proliferated in a cell-sphere form, and fibroblasts can inhibit proliferation of activated PBMCs so that PBMC cells cannot undergo cell-sphere-like proliferation. Since MSC (mesenchymal stem cell) were considered to have the ability to inhibit PBMCs proliferation, MSC cells were used as a positive control group.

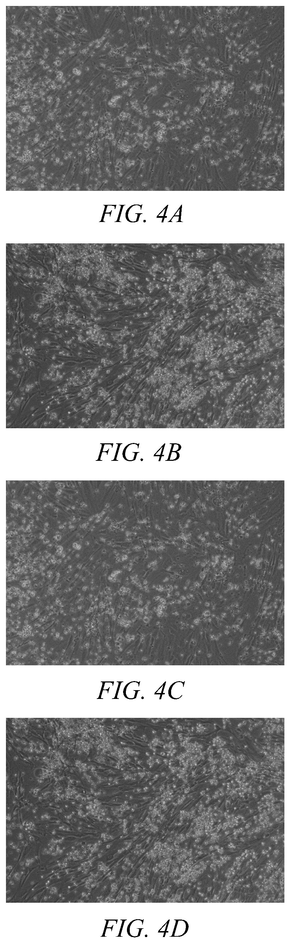

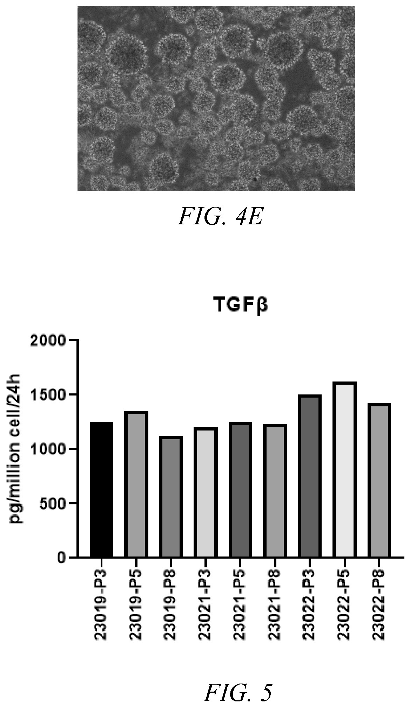

The results are shown in FIG. 4A to FIG. 4E. FIG. 4E shows the positive control (PBMC activation control) group, in which PBMC cells underwent spheroid-like proliferation; FIG. 4D shows the negative (PBMCs unactivated control) group, in which PBMC cells did not proliferate; FIG. 4A shows the 23019-PBMCs group, in which donor 23019 cells were co-cultured with activated PBMC cells, and PBMC cells exhibited a non-proliferative state as in the unactivated PBMC cells group; FIG. 4B shows the 23021-PBMCs group, in which donor 23021 cells were co-cultured with activated PBMC cells, and PBMC cells exhibited a non-proliferative state as in the unactivated PBMC cells group; FIG. 4C shows the MSC-PBMCs group, in which MSC cells were co-cultured with activated PBMC cells, and PBMC cells exhibited a non-proliferative state as in the unactivated PBMC cells group.

### 3. Secretion of transforming growth factor (TGFβ) by fibroblasts

After culture supernatants of fibroblasts derived from three donors at different passages were collected, a TGFβ detection kit was used to detect the content of TGFβ in the supernatants, and the content of TGFβ secreted per 1 million cells in 24 hours was calculated.

The results are shown in FIG. 5, from which it can be known that fibroblasts derived from different donors can all maintain a relatively high TGFβ secretion level, and this did not significantly change with cell passage. TGF-β can inhibit proliferation and differentiation of Th1, Th2 and CTL, thereby inhibiting a local inflammatory response; in cell experiments, it can inhibit PBMC proliferation, and in animal experiments, it can treat knee osteoarthritis.

### 4. Secretion of human stromal cell-derived factor (CXCL12) by fibroblast

After culture supernatants of fibroblasts derived from three donors at different passages were collected, a CXCL12 detection kit was used to detect the content of CXCL12 in the supernatants, and the content of CXCL12 secreted per 1 million cells in 24 hours was calculated.

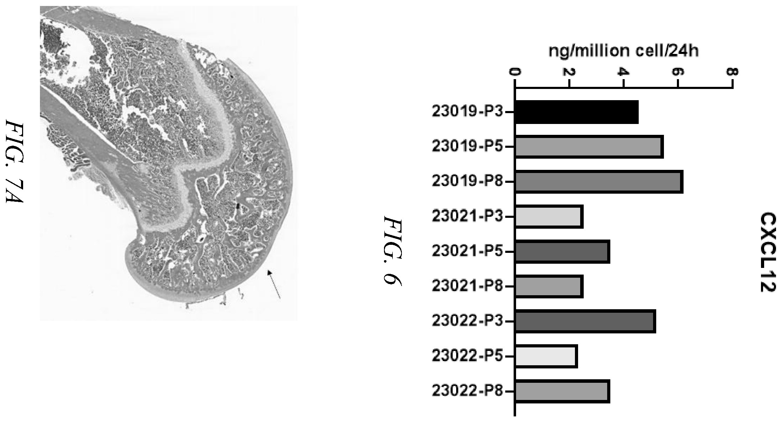

The results are shown in FIG. 6, from which it can be known that fibroblasts derived from different donors can all maintain a relatively high CXCL12 secretion level, and this did not significantly change with cell passage. Although a low concentration of CXCL12 can recruit immune cells, a high concentration of CXCL12 can have a repulsive effect on CD4+ and CD8+ T cells and inhibit local enrichment of T cells, thereby alleviating a local immune response; in cell experiments, it can inhibit PBMCs proliferation, and in animal experiments, it can treat knee osteoarthritis.

### Example 4. Application of fibroblasts

### 1. Osteoarthritis modelling method

Male SD rats aged 9-10 weeks and weighing 250-300 g were selected as research subjects; after the rats were anesthetized with isoflurane, the knee joint was shaved, and the rats were fixed in a supine position on a surgical board and continuously anesthetized. The knee joint was disinfected, the skin and muscle at the right knee joint site were sequentially incised, the knee joint was freed, and transection of the anterior cruciate ligament of the knee joint was performed. Then, the muscle and skin were sutured, wiped and disinfected with iodophor, placed on a heating pad for rewarming, and returned to a cage for feeding after awakening. The cruciate ligament transection group of the knee joint of rats was a model group, the untreated knee joint is a normal group, and the group in which cruciate ligament transection was performed on the knee joint and fibroblasts obtained in Example 2 are injected is an experimental group.

### 2. Cell injection

Two weeks after surgery, the test article (fibroblasts) was taken out from a liquid nitrogen tank and transported on dry ice; The test article was immediately immersed in a 37°C water bath and gently shaken until the liquid was completely melted, and was shaken left and right to mix uniformly. All the liquid was taken out with a pipette, added into a 50 mL sterile centrifuge tube, and centrifuged in a centrifuge set at 200 g for 5 minutes. After centrifugation was completed, the supernatant was discarded, and the cells were diluted to 2E+07/mL with normal saline. Using a microinjection needle, intra-articular injection of 1E+06 cells (50 microliters) was performed, wherein the knee joint injected with the test article was the experimental group.

### 3. Data collection

After the rats were euthanized with CO₂ 28 days after the first treatment, the right knee joint and the left knee joint were respectively dissected and collected, and sent to a third party for HE staining.

### 4. Experimental results

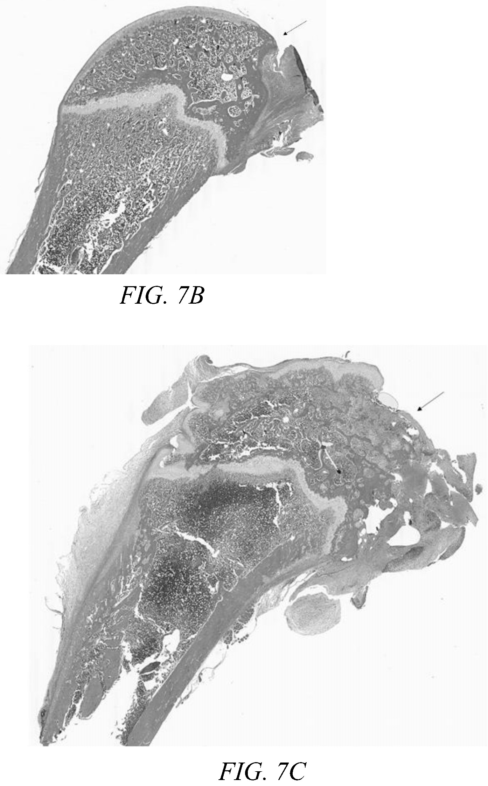

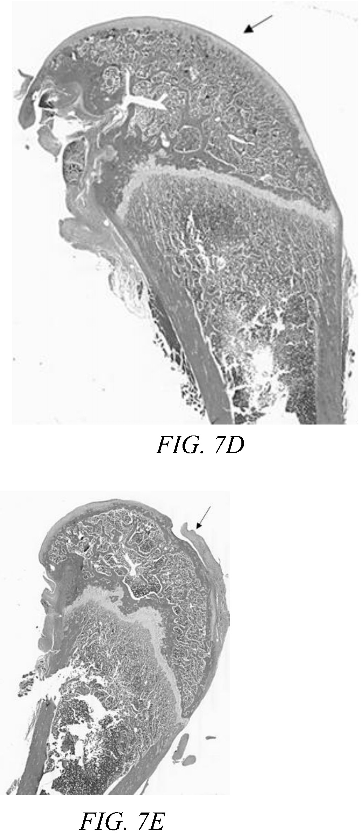

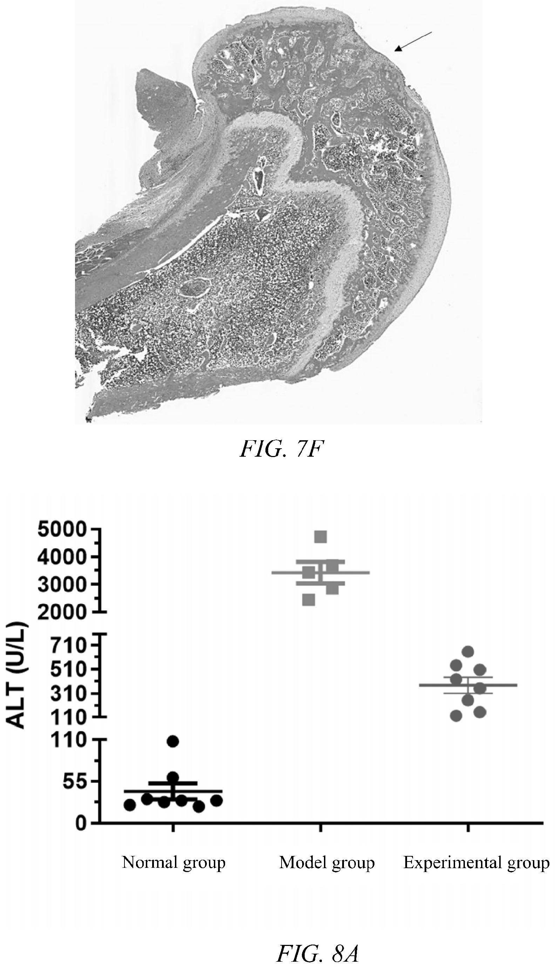

As shown in FIG. 7A to FIG. 7F, FIG. 7A and FIG. 7D respectively show staining of left and right knee joint tissues in the normal group, in which the joint sites were relatively smooth and had no damage; FIG. 7B and FIG. 7E respectively show staining of left and right knee joint tissues in the experimental group, in which damage existed at the joint sites; however, compared with the modelling group in FIG. 7C and FIG. 7F, in the experimental group injected with fibroblasts, the damage at the joint sites was relatively well repaired, and fibroblasts can relatively well repair knee osteoarthritis caused by ligament transection.

### 5. Method for acute-on-chronic liver failure modelling

Male SD rats aged 9-10 weeks and weighing 250-300 g were selected as research subjects; 10% CCL4 was injected into the abdominal cavity of the rats twice per week at 2 mL/kg for 12 consecutive weeks; in week 13, LPS was also administered by intraperitoneal injection at 1 mg/kg; the modelling group was not treated, and the experimental group was injected with fibroblasts obtained in Example 2 after injection of LPS.

### 6. Cell injection

The test article (fibroblasts) was taken out from a liquid nitrogen tank and transported on dry ice; The test article was immediately immersed in a 37°C water bath and gently shaken until the liquid was completely melted, and was shaken left and right to mix uniformly. All the liquid was taken out with a pipette, added into a 50 mL sterile centrifuge tube, and centrifuged in a centrifuge set at 200 g for 5 minutes. After centrifugation was completed, the supernatant was discarded, and the cells were diluted to 2E+07/mL with normal saline. After the rats were anesthetized with isoflurane, the rats were fixed in a supine position on a surgical board and continuously anesthetized; the hepatic portal vein site was freed, and intra-hepatic portal vein injection of 1E+07 cells (1000 microliters) was performed using an injection needle.

### 7. Data collection

After the rats were euthanized with CO₂ 3 days after the first treatment, liver tissues were respectively dissected and collected, sent to a third party for HE staining, and blood was collected for aminotransferase detection.

### 8. Experimental results

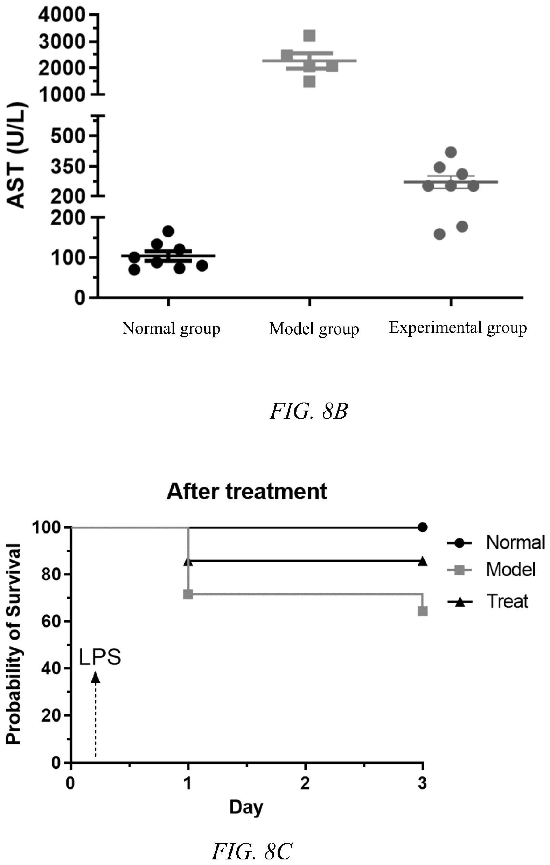

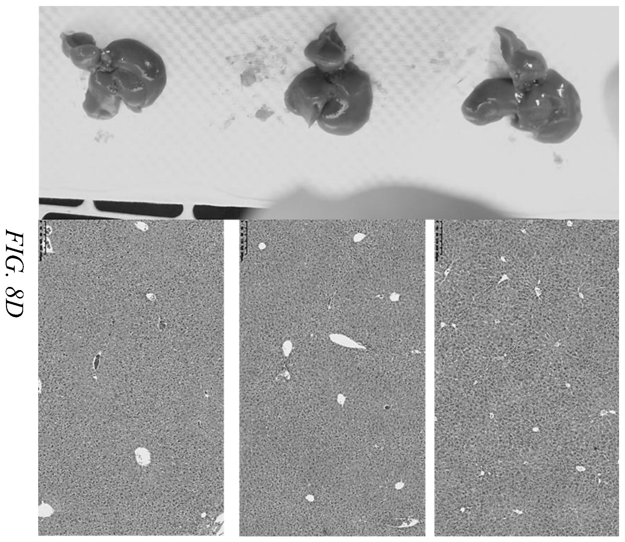

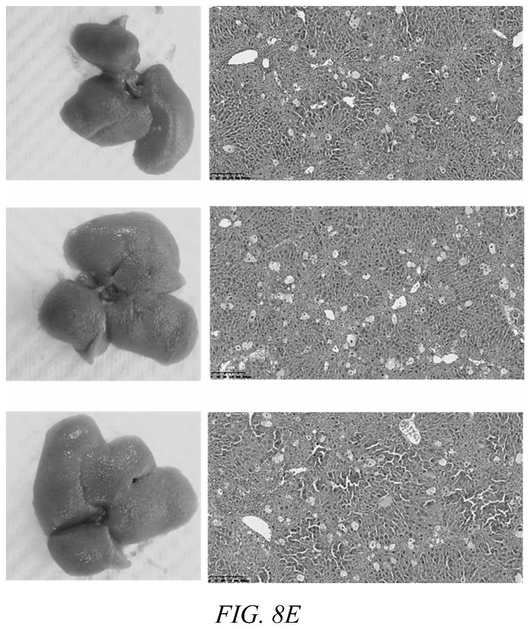

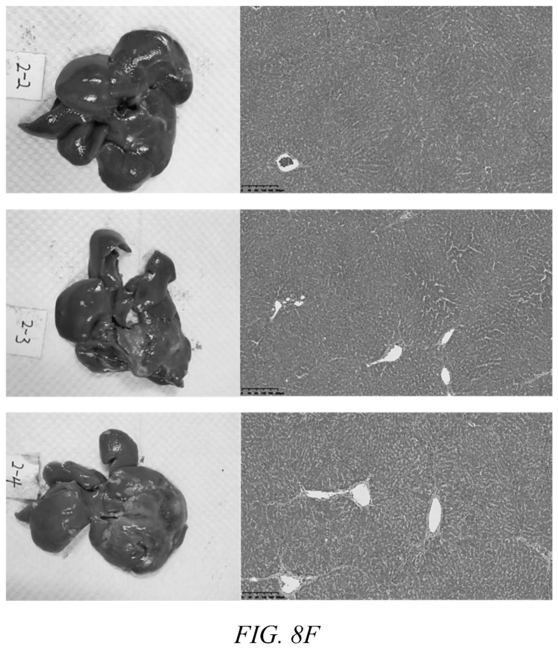

As shown in FIG. 8A to FIG. 8F, the normal group was male rats, the modelling group was male rats modelled with CCL4 and LPS, and the experimental group was a group injected with fibroblasts after modelling with CCL4 and LPS; FIG. 8A shows glutamic-pyruvic transaminase detection results after blood collection, wherein glutamic-pyruvic transaminase in the modelling group was significantly increased, and glutamic-pyruvic transaminase in the experimental group was obviously decreased and close to the value of the normal group; FIG. 8B shows glutamic-oxaloacetic transaminase detection results after blood collection, wherein glutamic-oxaloacetic transaminase in the modelling group was significantly increased, and glutamic-oxaloacetic transaminase in the experimental group was obviously decreased and close to the value of the normal group; FIG. 8C shows survival curves of animals in the three groups, wherein the initial number in each group was 10; no animals died in the normal group, 5 animals died in the modelling group, and 2 animals died in the experimental group; FIG. 8D shows pathological staining results after liver sampling from animals in the normal group; FIG. 8E shows pathological staining results after liver sampling from animals in the modelling group; FIG. 8F shows pathological staining results after liver sampling from animals in the experimental group.

## Claims

1. A method for preparing a fibroblast, **characterized in that** the method comprises the following steps:
S1: using a foreskin tissue as a raw material, and obtaining a primary fibroblast from a cell isolated by digestion with clostridiopeptidase A;
S2: performing in vitro culture of the primary fibroblast using a serum-free medium; wherein the serum-free medium is high-glucose DMEM supplemented with hydrocortisone, bFGF and human platelet lysate;
S3: obtaining the fibroblast, wherein the fibroblast shows positive expression of CD105, CD90, CD73, COL1A1 and FAP, with expression rates all exceeding 80%, negative expression of CD36, CD39, CD45 and HLA class II molecules, with expression rates below 5%, and can secrete TGF-β.

2. The method according to claim 1, **characterized in that** the foreskin tissue is a foreskin tissue discarded from clinical circumcision surgery.

3. The method according to claim 2, **characterized in that** the foreskin tissue is a surgical sample derived from a male patient not more than 16 years of age, and the patient has no infectious viral infection as determined by medical examination.

4. The method according to claim 1, **characterized in that**
the method of S1 is as follows:
S1-1: washing and disinfecting the foreskin tissue with normal saline, then adding neutral protease A, incubating and then washing the foreskin tissue with pre-cooled normal saline, mechanically separating the dermal layer and the epidermal layer of the tissue, removing the epidermal layer, cutting the dermal layer into pieces as finely as possible, washing the pieces with pre-cooled normal saline, and adding clostridiopeptidase A and incubating same to obtain the primary fibroblast;
S1-2: screen sorting the cell suspension obtained in S1-1l through a 70 µm cell strainer with the aid of normal saline, collecting the cell suspension under the cell strainer, centrifuging to discard the supernatant, washing by centrifugation with serum-free medium, and resuspending in the serum-free medium to obtain a primary cell suspension;
S1-3: screen sorting the primary cell suspension through a 40 µm cell strainer with the aid of normal saline, collecting the cell suspension harvested from filtration through the cell strainer to complete the screen sorting, centrifuging the resulting filtrate and discarding the supernatant to obtain the primary fibroblast.

5. The method according to claim 4, **characterized in that** the method of S1 is as follows:
S1-1: washing and disinfecting the foreskin tissue with normal saline, adding neutral protease A to a final concentration of 2 DMC U/mL, incubating the foreskin tissue at 37°C for 16-20 h, washing the foreskin tissue 1-2 times with pre-cooled normal saline, separating the dermal layer and the epidermal layer of the tissue using sterilized forceps, removing the epidermal layer, cutting the dermal layer as finely as possible into 0.5-2 mm pieces, washing the pieces 1-2 times with pre-cooled normal saline, transferring the pieces to a 50 ml centrifuge tube, adding clostridiopeptidase A to a final concentration of 0.1 PZ U/mL, and incubating the pieces at 37°C for 2-3 h;
S1-2: screen sorting the cell suspension through a 70 µm cell strainer with the aid of normal saline, collecting the cell suspension under the cell strainer, centrifuging at 1000 rpm for 5 minutes, discarding the supernatant, washing 1-2 times by centrifugation with serum-free medium, and resuspending in the serum-free medium to obtain the primary cell suspension.

6. The method according to claim 1, **characterized in that**
the method of S2 is as follows:
expanding the primary fibroblast in the serum-free medium until the cell confluence reached not less than 80%, then digesting the primary fibroblast with trypsin digestion solution, and subsequently subculturing the primary fibroblast using the serum-free medium to obtain the fibroblast.

7. The method according to claim 6, **characterized in that**
the method of S2 is as follows: digesting the primary fibroblast with the trypsin digestion solution for 1-5 minutes; the concentration of hydrocortisone is 5-25 ng/mL, the concentration of bFGF is 5-30 ng/mL, and the concentration of human platelet lysate is 4-10%; preferably, the concentration of hydrocortisone is 10-20 ng/mL, the concentration of bFGF is 15-25 ng/mL, and the concentration of human platelet lysate is 6-9%.

8. A fibroblast obtained by the method according to any one of claims 1 to 7, **characterized in that** the fibroblast shows positive expression of CD105, CD90, CD73, COL1A1 and FAP, with expression rates all exceeding 80%, negative expression of CD36, CD39, CD45 and HLA class II molecules, with expression rates below 5%, and can secrete TGF-β.

9. The fibroblast according to claim 8 for use in the preparation of a medicament for treating an inflammatory disease, preferably the inflammatory disease is local inflammation of osteoarthritis or acute liver failure.

10. The use according to claim 9, **characterized in that** the disease is osteoarthritis or acute liver failure.

11. A pharmaceutical composition, **characterized in that** the pharmaceutical composition comprises the fibroblast according to claim 8 as an active ingredient.

12. The pharmaceutical composition according to claim 11, **characterized in that** the pharmaceutical composition is a dosage form suitable for injection.

13. The pharmaceutical composition according to claim 12, **characterized in that** the pharmaceutical composition is a dosage form suitable for intra-articular injection, intramuscular injection and intravenous injection.
